# EUROPEAN PATENT APPLICATION

(11) **EP 1 726 322 A1**
(43) Date of publication of application: **29.11.2006**
(21) Application number: 05104409.7
(22) Date of filing: 24.05.2005
(51) Int. Cl.: A61M 15/00

(54) **Dose counter device for inhaler**

(71) Applicant: SHL Medical AB, 131 28 Nacka Strand (SE)
(72) Inventor: Brunnberg, Lennart, 135 49 Tyresö (SE); Olson, Stephan, 115 32 Stockholm (SE); Wieselblad, Anders, 113 64, Stockholm (SE)
(74) Representative: Holmberg, Nils Anders Patrik

(57) **Abstract**

The invention refers to a dose counter device for an inhaler (1) that in a reliable way will register a delivered dose from a canister (6) comprised in the inhaler, and that at the same time substantially will reduce the risk of falsely register a dose not delivered. Thus, the present invention will in an effective way, substantially avoid miscalculations of delivered doses from the canister.

## Description

### Technical field

The invention refers to a dose counter device for an inhaler that in a reliable way will register a delivered dose from a canister comprised in the inhaler, and that at the same time substantially will reduce the risk of falsely register a dose not delivered. Thus, the present invention will in an effective way, substantially avoid miscalculations of delivered doses from the canister.

### Prior art

Within the field of inhalers, dose counters are known that will count the number of doses delivered from a canister comprised in the inhaler. The user will thus for instance know, the number of doses taken or the numbers of doses remaining in the canister. A problem with known dose counters, is that they at times will register a delivered dose that never was delivered, and that they also may miss to register a dose that in fact was delivered. The user of the inhaler is thus provided with false information about the number of doses remaining in the inhaler, which may constitute a major problem for for instance an asthmatic person which thus unintentionally may run out of medicament.

Therefore, there is a general need for a dose counter device that in a reliable way will register a delivered dose from a canister comprised in the inhaler, and that at the same time substantially will reduce the risk of falsely register a dose not delivered.

### Disclosure of the invention

The object of the present invention is therefore to provide a dose counter device that satisfies the above described need.

### Brief description of the drawings

Fig. 1 illustrates a general inhaler comprising a liquid medicament containing canister, when the inhaler is in a non-activated state,
Fig. 2 illustrates a dose counter device according to a first preferred embodiment of the invention (not to scale),
Fig. 3 illustrates a calibrating member of the second embodiment,
Fig. 4 illustrates the calibrating member of figure 3, mounted on top of the inhaler,
Fig. 5a illustrates a driving member of the second embodiment,
Fig. 5b illustrates a house of the second embodiment,
Fig. 6 illustrates the components of fig.3-5 mounted on top of an inhaler,
Fig. 7 illustrates the distal end of the dose counter device according to the second embodiment mounted on top of an inhaler in cross-section seen from above,
Fig. 8 illustrates the force applied to the distal end of the canister as a function of the distance that the canister body has moved towards the proximal end of the canister,
Fig. 9 illustrates an embodiment of the third preferred embodiment of the invention (not to scale).

### Detailed description of the invention

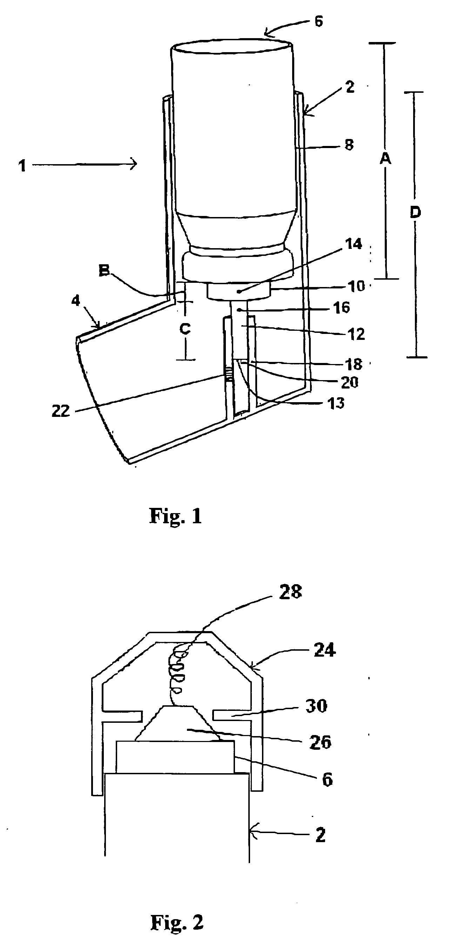

With reference to figure 1, a general inhaler 1 comprises a housing 2 having a mouthpiece 4, which the user puts in his mouth when a dose of medicament is to be inhaled. The housing 2 of the inhaler is adapted to receive a standard canister 6, containing liquid medicament, wherein the distal end of the canister 6 protrude a certain distance from the distal end of the housing 2.
The canister comprises a main canister body 8 that is adapted to communicate with a dose chamber 10. The dose chamber 10 is in turn provided with a hollow spring-suspended transfer tube 12 provided with an outlet 13 in its proximal end. The dose chamber is further provided with an outlet valve 14 that is adapted to correspond to a valve 16 in the transfer tube 12.
The interior of the mouthpiece 4 is provided with a tubular receiving member 18, having an inward protruding flange 20, provided a predetermined distance from the bottom of the receiving member 18. The receiving member 18 is further provided with an outlet 22 that communicates with the outlet of the mouthpiece 4.
The proximal end of the transfer tube 12 abuts against the flange 20, such that a part of the outlet 13 of the transfer tube communicates with the outlet 22 of the receiving member.

When a user of the inhaler intends to inhale a dose, he puts the mouthpiece in his mouth and applies a force, generally by the aid of his hand and fingers or the like, on the distal surface of the canister 6, such that the canister body 8 and the dose chamber 10 is forced downwards towards the bottom of the housing 2, i.e. towards the proximal end of the canister, while the transfer tube 12 remains still. Thus, when the dose chamber 10 has moved a predetermined distance towards the bottom of the housing, the valve 16 of the transfer tube 12 will open communication with the valve 14 of the dose chamber, such that a metered dose of the pressurised liquid medicament contained in the main body 8 will flow from the dose chamber 10, through the valves 14, 16, the transfer tube 12, the outlets 13, 22 and out through the outlet of the mouthpiece 4. When the user releases the force applied to the distal end of the canister, it will move back to its original position.

The distance between the valves 14, 16 is a known predetermined distance, generally 2 mm, when the inhaler is in the first non activated state, i.e. the dose chamber needs to in a second activated state be forced downwards with a distance of 2 mm for the valves 14, 16 to open communication with each other. It would be a simple case to construe a reliable dose counter device if the dimensions of the inhaler, would be exact dimensions. However, all dimensions of the general inhaler, such as the height of the main body A, the height of the dose chamber B, the length of the transfer tube C, and the distance between the proximal end of the transfer tube and the distal edge of the housing D, are impaired by variation of not neglectable magnitude. If for instance the variation of the distances A, B, C and D is ±0.5 mm, ±0.05 mm, ±0.25 mm and ±0.2 mm, respectively, the sum of all margin of errors will be ± 1 mm.
So, if one for instance construes a dose counter device that determines the distance that the canister has moved towards the bottom of the housing, dependent on a single reference point, for instance the distal edge of the housing 2, and register a delivered dose when said distance amount to 2 mm, the dose may or may not have been delivered. Also, the dose may have been delivered without the distance amounting to 2 mm.

One solution to this problem, is according to the present invention an all mechanic solution and refers to provide for a dose counter device that connect one reference point with a second reference point, so that the movement of said reference points towards the proximal end of the canister can be determined in a reliable way. The inventive dose counter device is provided as a non-disposable article, preferably mounted on top of the inhaler, and is thus adapted to be used repeatedly with several numbers of canisters. That is, when one canister is empty, the user may through away the used canister, mount a fresh canister in the housing and once again mount the dose counter device on top of the inhaler for new use. The counter may also be construed for being disposable, still facilitating removal and reattachment to allow for cleaning of the inhaler and its nozzle.

In figure 2, a dose counter device according to the first embodiment is illustrated. The dose counter device comprises a house 24, which distal part preferably is conically shaped. The inner diameter of the tubular proximal part of the house 24 is slightly larger than the outer diameter of the housing 2 of the inhaler, such that the house 24 can be tightly, but movably mounted on top of the inhaler. In the interior of the house 24, a spring-suspended cone 26 is provided with its base facing towards the proximal end of the inhaler, wherein a helical spring 28 operates between the distal end of the cone 26 and the inner distal end of the house, as seen in figure 2. The base of the cone 26, i.e. the proximal surface of the cone 26, is adapted to abut against the distal surface of the canister 6. The interior of the house 24 is further provided with inward protruding protrusions 30, equally distributed along the inner circumference of the house 24. The number of the protrusions 30 are preferably 2-4.

So, the dose counter device illustrated in figure 2, is mounted on top of the inhaler so that the base of the cone is in contact with the distal surface of the canister. This constitutes the first reference point, as described above. The cone 26, thus serves as a first reference point establishing member. It is to be understood that said member can have other configurations than the cone shown in figure 2. When the user intends to inhale a medicament dose, he instead of applying a force directly on the distal end of the canister 6, applies a force on the distal end of the house 24, such that the house moves down towards the proximal end of the inhaler. Due to the spring 28, the cone 26 and the canister 6 lie still until the protrusions 30, which are moved down towards the proximal end of the inhaler along with the house 24, come in contact with the cone 26. The cone 26 will thus move along down towards the proximal end of the inhaler as soon as the protrusions abut against the cone 26. When the protrusions come in contact with the cone 26, this constitute the second reference point described above and the dose counter device is thus provided with means that determines when the protrusions have moved a distance of 2mm down towards the proximal end of the canister from the second reference point in relation to a fix third reference point of the inhaler, preferably the distal edge of the inhaler housing. The protrusions 30 thus serves as a second reference point establishing member. It is to be understood that said member can have other configurations than the above described.

Thus, when said distance amount to 2 mm, the dose counter device register a delivered dose. The register of a delivered dose can be accomplished and visualized for the user in a number of ways. The exterior of the housing can for instance be provided with a threaded plunger provided with a cursor that moves one step when a dose delivery is registered, or a toothed wheel can be provided in the interior of the house that is rotated one step when the dose delivery is registered together with a second or further wheel(s) that are driven one step per full turn of the respective preceding decimal wheel.

When the user of the inhaler releases the force applied to the house 24, the canister will move back to its original position and in turn the accumulated energy in the spring 28, will urge the house back to its original position.

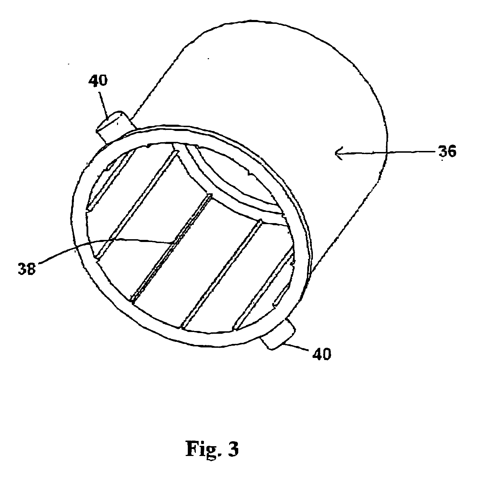

A second preferred embodiment of the present invention is also an all mechanical solution. In this second embodiment a fixed first reference point is established during the manufacturing of the dose counter device. In figure 3 is shown a calibrating member 36 having a sleeve-formed configuration. The interior of the calibrating member has inward preferably equally distributed protruding ribs 38 extending along the longitudinal axis of the member 36. The ribs 38 preferably has a triangular shape and are manufactured of an easily deformable plastic material. The outer surface of the proximal end of the member 36 is provided with preferably two equally distributed outward protruding pins 40.

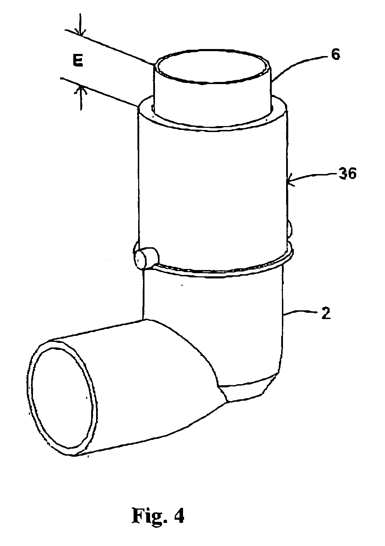

The calibrating member 36 is adapted to be mounted over the distal end of the inhaler housing 2, as seen in figure 4. The ribs 38 will during this mounting procedure deform and break and the calibrating member will thus be tightly and not removably fitted on the inhaler housing 2. This mounting procedure is a well controlled process so that the distance between the distal end of the calibrating member 36 and the distal end of the canister 6, when the mounting procedure is finished, is a well defined and predetermined distance, indicated in figure 4 by the letter E.

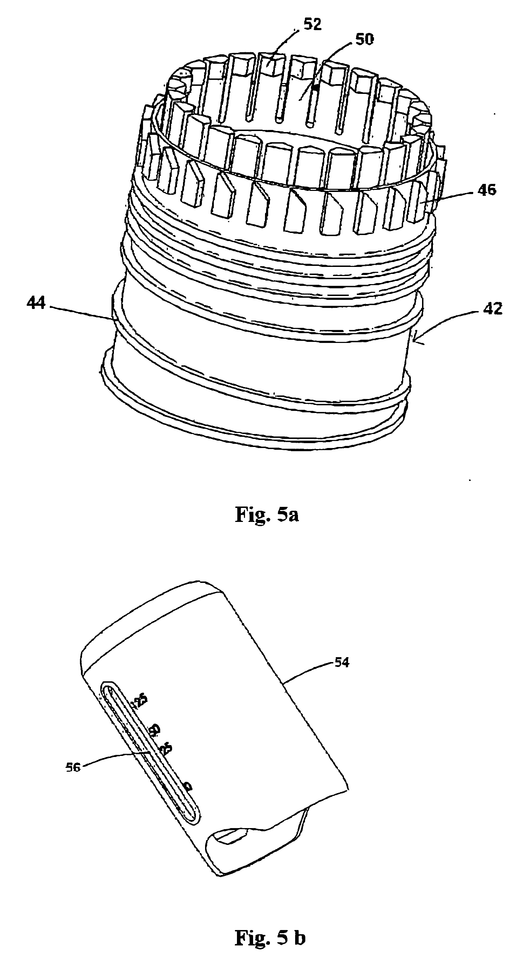

In figure 5a and 5b is shown two further components adapted to be used in the dose counter device of the second embodiment. One of these components is a driving member 42 having a sleeve-formed configuration designed in accordance with the calibrating member. The interior surface of the member 42 is at its proximal end provided with a means (not shown) that corresponds to a means (not shown) on the exterior surface at the proximal end of the calibrating member, so that the driving member is adapted to be fitted over the calibrating member and there adapted to be rotated but not be movable along the longitudinal axis of the calibrating member.
The exterior surface is further provided with a thread 44, preferably having a variable pitch of grooving. As seen in figure 5a, the pitch of grooving is more fine towards the distal end of the driving member. The exterior surface of the distal end of the driving member is further provided with a number of equally distributed pins 46 having a bevelled edge at their distal end. The driving member is further at its distal end provided with upward protruding pins 50. The pins 50 are at their distal end provided with triangulary shaped teeth 52 protruding towards the interior of the driving member.

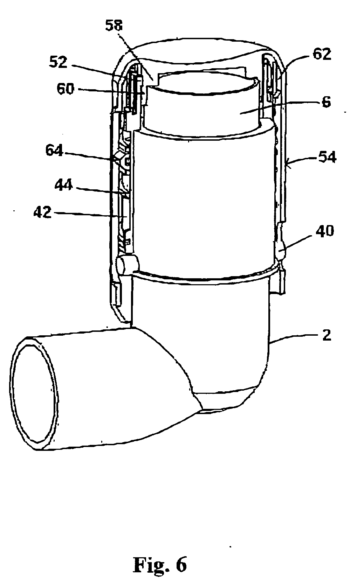

The driving member is adapted to be fitted in the interior of a house 54. The house is provided with a dose window 56, and means that corresponds to the pins 40 of the calibrating member. In order for the driving member to be fitted in the house 54, the inner surface of the distal end of the house is thus provided with a means that corresponds to the means provided on the distal end of the driving member.
So when the dose counter device according to the second embodiment is to be used, the driving member is mounted in the interior of the house 54, and the two components are mounted over the calibrating member already fitted with the inhaler housing, see figure 6.

In figure 6 it is shown that the interior surface at the distal end of the house 54 is provided with a downward protruding flange 58, adapted to be in contact with the distal end of the canister 6. That is, when the dose counter is mounted and ready for use, the distance between the distal end of the calibrating member and the distal end of the canister 6, i.e. the proximal edge of the flange 58 and the distal end of the calibrating member is a known predetermined distance.

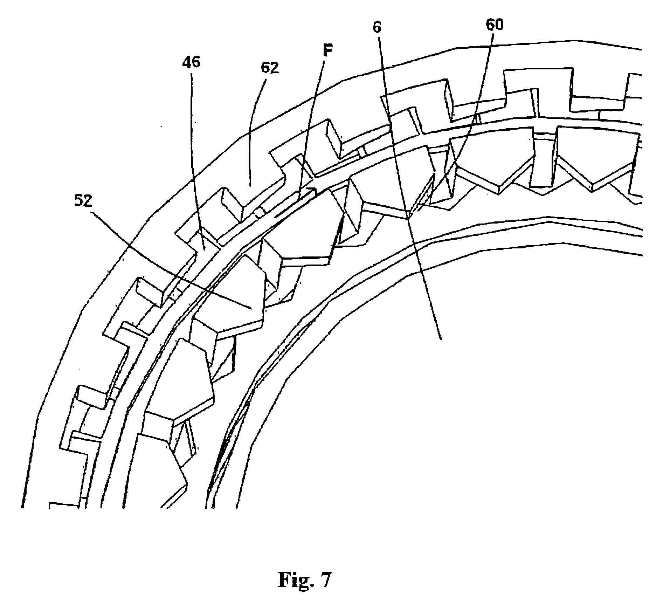

When the user of the inhaler intends to inhale a dose, he applies a force on the distal end of the house 54, which thus moves towards the proximal end of the canister. The flange 58 will thus urge the canister downwards. The flange 58 is further provided with teeth 60, that correspond to the teeth 52 of the driving member, as seen in figure 6 and in figure 7, which illustrates a cross section of the distal end of the dose counter device when mounted on top of an inhaler. The interior surface of the distal end of the house is further provided with inward protruding pins 62 having a bevelled edge at their proximal end that correspond to the bevelled edge of the pins 46 on the driving member.
When thus the house 54 is urged downwards, the bevelled edge of the pins 62 will move along the bevelled edge of the pins 46 and urge the driving member to rotate in direction indicated by the arrow F in figure 7. Since the height between the proximal end of the flange 58 and the distal end of the calibrating member, the distance that one tooth 52 have to move in order to for its tip to meet the tip of a tooth 60 of the house 54 is thus a known predetermined distance. When a tooth 52 slides over a corresponding tooth 60, the dose counter device will register a delivered dose.

The driving member is thus rotated one step when the house is applied with a force on its distal end that will urge a tooth 52 to slide over a tooth 60. A dose indicating member 64, is provided over the thread 44 but provided in the dose window 56. The member 64 thus rides along the thread when the driving member is rotated and moves down towards the proximal end of the dose window, indicating the dose remaining in the canister. In figure 5b, the dose scale is seen provided as a progressive scale that corresponds to the variable pitch of grooving of the thread, but naturally the thread can have a non-variable pitch of a grooving, that correspond to a non-progressive dose scale.

Preferably spring means (not shown) is provided between the inner distal end of the house and the distal end of the canister so that the house springs back towards the distal end of the inhaler when the user releases the force applied to the distal end of the house.

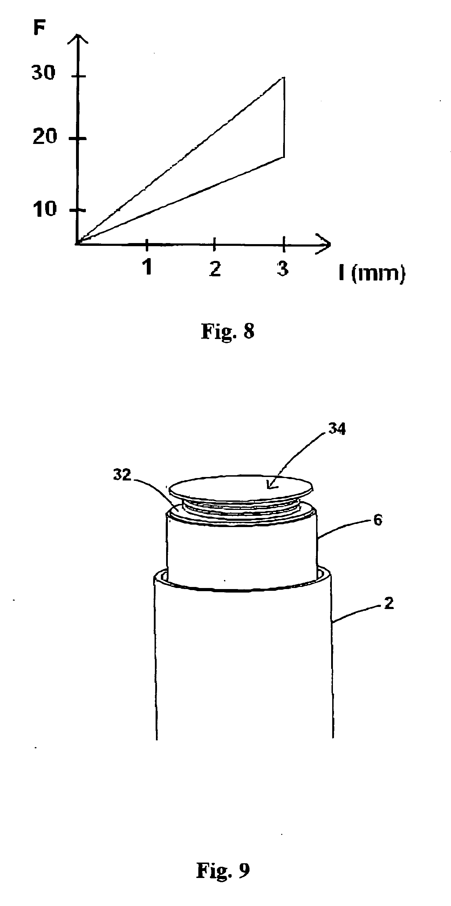

According to a third embodiment of the present invention, the dose counter device of the present invention, utilizes the force that needs to be applied on the distal end of the canister in order to move the canister body down towards the proximal end of the canister. Figure 8 describes schematically in a diagram, the force that is applied to the distal end of the canister as a function of the distance that the canister body has moved towards the proximal end of the canister. As seen, the force-way-diagram describes a hysteresis-type of curve, and the force that needs to be applied to the distal end of the canister in order to urge the canister body a distance of 2 mm is approximately 20 N. Naturally, this force-way-diagram may differ from one canister to another.

According to the third embodiment, the distal end of the canister is preferably provided with plate 32 of a conducting material, such as for instance a brass plate. The brass plate is in turn provided with pressure sensing means, such as for instance a strain gauge or a piezo element, that will sense the force value applied to the brass plate, that is on the distal end of the canister. When the force value amount to a certain value, for instance 10 N that in the force-way-diagram of figure 8, corresponds to a movement of the canister body towards the proximal end of the canister with a distance of 1 mm, the pressure sensing means will send a signal to an electronic circuit with information to an acoustic sensing means comprised in the circuit, provided on the distal end of the canister, to start to listen after sound. The delivery of a dose from the canister will namely generate a sound that will propagate to the distal end of the canister and which can be registered by the acoustic sensing means as a delivered dose. The pressure sensing means is thus adapted to send a signal which activates the acoustic sensing means as long as the force value applied to the distal end of the canister, i.e. the force value sensed by the pressure sensing means, is above or equal to 10 N, i.e. the acoustic sensing means is adapted to operate within a predetermined range. This will minimize the risk of the acoustic sensing means registering a sound that does not has its origin in the delivery of a dose. When the force applied by the user to the brass plate amounts to 10 N, one can be certain that the user intends to inhale a dose and that the sound of the dose delivery is soon to follow.

Preferably the acoustic sensing means is provided with means, such as a signal interpreting means, so that the acoustic sensing means is adapted to register a sound as a delivered dose, only when it picks up a sound that corresponds to the sound that has its origin from the delivery of a dose. The delivery of a dose from the canister, namely generates a characteristic sound that can be identifiable by means of the signal interpreting means. Said interpreting means can for instance be provided with means that compares the spectra of the picked up sound with the spectra of the sound of a delivered dose. If there is a mismatch between said spectra, a sound is not registered as a dose delivery since the sound picked up thus had its origin from something else.

In order to minimize the power need of the dose counter device, said device can be provided with means that close the electronic circuit provided on the distal end of the canister only when a certain force is applied to said canister. Moreover, if a piezo element is used as the pressure sensing means, said element when applied with a force, will generate an electric voltage that will activate the electronic circuit. That is, when the inhaler is not in use, the electronic circuit is not closed and does not consume any energy. However, the dose counter device is preferably provided with means in order to be connected to an external power source, such as a battery, even though it might be possible for the piezo element to be the only power source needed to operate the device.

Preferably the pressure sensing means is also used as the acoustic sensing means. For instance, if a strain gauge is used as the pressure sensing means, a at least one further strain gauge can be provided on the distal end of the canister, which strain gauge is used as the acoustic sensing means as described above. It is also feasible that the strain gauge that serves as the pressure sensing means, is adapted to also have the function of being the acoustic sensing means. The same situation applies when the pressure sensing means is a piezo element. That is, the piezo element used as the pressure sensing means can also be used as the acoustic sensing means, or at least one further piezo element, provided on the distal end of the canister, can be used as the acoustic sensing means. Naturally, the use of a strain gauge as the pressure sensing means does not rule out the use of a piezo element as the acoustic sensing means, and vice versa. The components can thus be used in any combination. If a piezo element is used as the pressure sensing means and/or the acoustic sensing means, the dose counter device can be provided with spring means in order to reduce the flex of the piezo element in order to reduce of the risk for said element to break or crack.

The dose counter device can also be provided with spring-suspended means 34, such as a second conducting plate, spring-mounted over the distal end of the canister, as seen in figure 9, so that the force applied to the canister by the user when he intends to inhale a dose, instead of being applied directly on the plate 32 is applied on the second plate 34. In this case, the means for registering a sound, i.e. the pressure sensing means, the electronic circuit, the acoustic sensing means and the signal interpreting means if any, can be provided on any or both of said plates. When the two plates connect, the electronic circuit will close and the pressure sensing means will sense the force applied to distal end of the canister and send a signal which activates the acoustic sensing means, as described above. This embodiment will minimize the risk of registering false information.

The delivered dose and/or the doses remaining in the canister can be visualized for the user in a number of ways, such as through en electronic display provided in the inhaler or the like. Information about taken or remaining doses and e.g. time point may also be distributed by e.g. radiofrequency such as Bluetooth to another device where the information is displayed or used for compliance measuring, as described for instance in SE0300729-1.

## Claims

1. Dose counter device for an inhaler that registers when a dose is delivered, adapted to be mounted on the distal end of the inhaler (1), said inhaler (1) comprises a canister (6) comprised in an inhaler housing (2), wherein the distal end of the canister (6) protrudes a distance from the distal end of the inhaler housing (2), and wherein the canister (6) has a canister body (8) and further in its proximal end has a dose chamber (10) having a valve means (14) and a transfer tube (12) having a valve means (16), wherein the valve means (14; 16) are placed a predetermined distance from each other along the longitudinal axis of the canister (6) when the inhaler (1) is in a first non-activated state, and wherein the valves (14; 16) are adapted to communicate with each other when the distal end of the canister (6) in a second activated state is applied with a force that urges the canister chamber (10) over the transfer tube (12) towards the proximal end of the canister (6) a distance that is equal to said predetermined distance, which will expel medicament from the canister (6) **characterized in that** the dose counter device is provided with means that determines when the dose body (8), and thus the dose chamber (10) and the valve means (14), have moved the predetermined distance towards the proximal end of the canister and then registers a delivered dose.

2. Dose counter device according to claim 1, **characterized in that** the means that determines when the canister body (8) has moved the predetermined distance towards the proximal end of the canister (6), comprises means that will establish a first reference point of the inhaler (1) when the inhaler is in the first non-activated state and that will establish a second reference point during a movement of the dose counter device towards the proximal end of the canister (6), and **in that** the dose counter device is provided with means that will determine when the first and the second reference point have moved the predetermined distance towards the proximal end of the canister (6) during the second activated state of the inhaler (1) in relation to a fix third reference point of the inhaler and then registers a delivered dose.

3. Dose counter device according to claim 2, **characterized in that** the third reference point is the distal edge of the inhaler housing (2).

4. Dose counter device according to any of claims 1-3 **characterized in that** the dose counter device comprises a house (24), the interior of said house (24) further comprising a first reference point establishing member (26) and a spring (28), the proximal end of the spring (28) is connected to the distal end of the first reference point establishing member (26) and the distal end of the spring is connected to the inner distal end of the house (24), wherein the proximal end of the first reference point establishing member (26) is adapted to be in contact with the distal end of the canister (6), which establishes the first reference point, and wherein the dose counter device is adapted to move towards the proximal end of the canister when applied with a force on its distal end, during which movement a second reference point establishing member (30) comes in contact with the first reference point establishing member (26) which establishes the second reference point.

5. Dose counter device according to claim 4 **characterized in that** the first reference point establishing member is a cone (26), wherein the base of said cone (26) constitutes its proximal end, and **in that** the second reference point establishing member is inward protruding protrusions (30) of the house (24).

6. Dose counter device according to claim 1 **characterized in that** the means that determines when the dose body (8) has moved the predetermined distance towards the proximal end of the canister, comprises pressure sensing means provided in an electronic circuit on the distal end of the canister, said pressure sensing means is adapted to determine the force value applied to the distal end of the canister during the second activated state of the inhaler.

7. Dose counter device according to claim 6 **characterized in that** the electronic circuit further comprises an acoustic sensing means also provided on the distal end of the canister, and **in that** the pressure sensing means is adapted to activate the acoustic sensing means when the force that is applied to the distal end of the canister is substantially equal to and/or above a predetermined force value, and **in that** the acoustic sensing means registers a dose delivery when it picks up a sound.

8. Dose counter device according to any of claims 6-7 **characterized in that** the distal end of the canister is provided with means of a conducting material.

9. Dose counter device according to any of claims 6-8 **characterized in that** the pressure sensing means is a strain gauge or a piezo element.

10. Dose counter device according to any of claims 6-9 **characterized in that** the acoustic sensing means is a strain gauge or a piezo element.

11. Dose counter device according to any of claims 6-10 **characterized in that** the pressure sensing means is also used as the acoustic sensing means.

12. Dose counter device according to any of claims 6-11 **characterized in that** the acoustic sensing means is provided with means that compares the spectra of a picked up sound with the spectra of the characteristic sound of a delivered dose and if there is a match between said spectra will register a sound as a dose delivery.
